# EUROPEAN PATENT APPLICATION

(11) **EP 4 155 384 A1**
(43) Date of publication of application: **29.03.2023**
(21) Application number: 21831827.7
(22) Date of filing: 24.06.2021
(51) Int. Cl.: C12N 5/00, C12N 5/071

(54) **METHOD FOR PREPARING COMPOSITION FOR CULTURING PANCREATIC ORGANOIDS, COMPOSITION THEREFOR, AND METHOD FOR CULTURING ORGANOIDS BY USING SAME**

(30) Priority: 30.06.2020 KR 20200080514; 30.04.2021 KR 20210056943
(71) Applicant: Gradiant Bio Convergence Cooperation, Seoul 05835 (KR); POSTECH Research and Business Development Foundation, Pohang-si, Gyeongsangbuk-do 37673 (KR)
(72) Inventor: LEE, Ha Ram, Seoul Seoul (KR); JANG, Jin Ah, Pohang-si Gyeongsangbuk-do 37834 (KR); CHOI, Yoo Mi, Pohang-si Gyeongsangbuk-do 37756 (KR); HWANG, Dong Gyu, Pohang-si, Gyeongsangbuk-do 37666 (KR); KIM, Myung Ji, Daegu 41586 (KR)
(74) Representative: Isarpatent
(86) International application number: PCT/KR2021/007963
(87) International publication number: WO 2022/005106

(57) **Abstract**

The present invention relates to a preparation method of a composition for culturing pancreatic organoids, a composition thereby, and an organoid culture method using the same. The present invention is capable of creating an environment that is more similar to an actual tissue than a conventional Matrigel-based culture system, and in particular, exhibits an effect of facilitating tissue differentiation in pancreatic organoid culture and effectively developing into a form that is similar to an actual tissue.

## Description

### TECHNICAL FIELD

The present invention relates to a preparation method of a composition for culturing pancreatic organoids, a composition thereby, and an organoid culture method using the same.

### BACKGROUND ART

Organoids are three-dimensional cell aggregates formed from stem cells through phylogeny and differentiation, and they allow for the observation of the development process of organs and the reproduction of actual tissues in terms of function and structure. As such, a support or scaffold, which plays a supporting role so that cells can be attached and grow, also plays a very important role in biological tissue engineering, and it also plays an important role in the growth of cells seeded in a porous structure and cells migrating from the surroundings of the tissue. Most of the cells in the human body are adherent cells that are attached to grow, and if there is no place to be attached to, the cells cannot grow and eventually die. Therefore, the scaffold should provide a compatible environment for cell adhesion, differentiation, growth and cell migration. Such a scaffold can be made of various materials, and research to develop a scaffold by using a natural material, a synthetic polymer, bioceramics, and a polymer-ceramic composite material is being actively conducted. Accordingly, organoids that mimic various organs have been developed so far, and these are mainly cultured in Matrigel, a three-dimensional culture environment.

However, although Matrigel is widely used for organoid culture, it is derived from mouse sarcoma, and there is no particular substitute. Therefore, despite the high cost, there is no choice but to rely on Matrigel. In addition, certain components are dominating in Matrigel and there is a limitation in reflecting the tissue-specific characteristics. To complement and replace this, decellularization of tissues and organs has been studied as a promising method for preparing functional scaffolds for cell culture and transplantation, and there is an emerging need for the method. However, the organoids produced by the current organoid culture technology have many differences from the actual human tissues in terms of the degree of differentiation and functions, and thus the development of a technology for culturing more mature organoids is required.

In this regard, Non-Patent Document 1 below teaches that a decellularized pancreatic tissue can be used as scaffold for pancreatic islet formation. However, the decellularization process is not specifically disclosed therein.

In addition, the Patent Document 1 below discloses a preparation method of a brain organoid culture composition in which brain tissue is decellularized and lyophilized, the lyophilized tissue is dispersed and then solutionized in an acidic solution, and the pH is adjusted. However, this is for brain tissue and not for the pancreatic. Moreover, it is only described about the solutionizing that pepsin or trypsin can be used as an acidic solution, and just the general gelation temperature (25 to 38°C) after pH adjustment is described.

Therefore, there always exists a need for research and development on a preparation method of a composition for culturing pancreatic organoids, which can increase the culture efficiency of pancreatic organoids, and a composition thereby and a organoid culture method using the same.

### [Prior Arts]

### [Patent Document]

(Patent Document 1) KR patent publication: 10-2019-0143830 (published on December 31, 2019)
(Patent Document 2) KR registered patent: 10-2015368
(Patent Document 3) KR patent publication: 10-2017-0078460

### [Non-Patent Document]

(Non-Patent Document 1) Damodran, RG. & Vermette, P. J Tissue Eng Regen med.

### DETAILED DESCRIPTION OF THE INVENTION

### TECHNICAL PROBLEM

The purpose of the present invention is to solve the problem that the Matrigel-based culture system, which is an extract derived from animal cancer tissue, has a large difference between batches, is incapable of simulating the environment of the actual tissue, and has an insufficient efficiency of tissue differentiation and development. The composition of the present invention is to provide an optimal environment for organoid culture by creating an actual tissue-like environment and to improve organ-specific differentiation potency.

In addition, another purpose of the present invention is to provide a composition for culturing an organoid, which has excellent physical properties of a decellularized tissue and which can also significantly increase the organoid culture efficiency, and a preparation method of the same.

In addition, another purpose of the present invention is to provide a composition for culturing an organoid that is capable of providing excellent physical properties of a decellularized tissue, preventing gelation, and enabling uniform hydrogelation, and a preparation method of the same.

### TECHNICAL SOLUTION

In order to achieve the purposes above, the present invention provides a preparation method of a composition for culturing pancreatic organoids, the composition thereby, and a organoid culture method using the same.

The present invention provides a preparation method of a composition for culturing pancreatic organoids, the method comprising: S1) decellularizing pancreatic tissue; S2) lyophilizing the decellularized tissue; S3) pulverizing the lyophilized tissue; S4) lysing the pulverized tissue by adding a proteopeptic enzyme and an acid; and S5) adjusting the pH by adding a basic solution to the lysate.

Since the decellularized tissue contains an actual tissue-specific extracellular matrix component, it is able to provide a physical, mechanical, and biochemical environment of the tissue, and is very efficient in promoting the differentiation into pancreatic tissue cells and their tissue-specific functionality.

The "organoid" refers to a micro-sized biological organ produced in the form of an artificial organ by culturing cells derived from tissues or pluripotent stem cells in a 3D form.

The organoids are three-dimensional tissue analogues including organ-specific cells that are developed from stem cells and that self-organize in a manner that is similar to the *in vivo* state, and they can develop into specific tissues by patterning limited factors (for example, growth factors).

The organoid has the intrinsic physiological properties of the cell and may have an anatomical structure that mimics the original state of a cell mixture (including not only limited cell types but also residual stem cells and proximal physiological niche). Through a three-dimensional culture method, the organoid may have a shape and a tissue-specific function, such as that of an organ in which cells and cellular functions are better arranged and which has functionality.

According to one embodiment of the present invention, the S1) decellularizing may comprise, twice or more, a process of adding at least one solution selected from sodium dodecyl sulfate (SDS), polyethylene glycol (PEG), Triton X-100, ethylenediaminetetraacetic acid (EDTA), peracetic acid, deoxycholic acid, 3-[(3-cholamidopropyl)dimethylammonio]-1-propanesulfonate (CHAPS), sodium deoxycholate, ammonium hydroxide, trypsin, benzonase, DNase, calcium chloride, magnesium sulfate, sodium azide (NaN₃) and sodium chloride. In addition, S1) may comprise a washing process in the middle of the two decellularization processes. In one embodiment, the tissue may be stirred during the decellularization.

Decellularized extracellular matrix (dECM) can be obtained through the method described above, and the "dECM" means a natural scaffold for cell growth prepared through decellularization of tissues found in mammals and multicellular organisms. The extracellular matrix may be collagen, elastin, laminins, glycosaminoglycans, proteoglycans, antimicrobials, chemoattractants, cytokines, and a mixture of structural and non-structural biomolecules that are not limited by growth factors. The extracellular matrix contain about 90% collagen in various forms in mammals. Extracellular matrixes derived from various biological tissues may have different overall structures and compositions due to the unique role required for each tissue.

According to one embodiment of the present invention, the decellularizing may comprise: a1) washing a pancreatic tissue; a2) treating with at least one selected from peracetic acid, triton-X, and SDS; a3) treating with at least one selected from ethanol, isopropanol and n-propanol; a4) washing; a5) treating with at least one selected from peracetic acid, triton-X, and sodium dodecyl sulfate; and a6) washing.

In addition, the washing of a1), a4), or a6) may be performed with at least one selected from distilled water, physiological saline, and phosphate buffered saline. When decellularizing is performed by the method described above, a composition for culturing organoids having excellent differentiation into tissue cells and having excellent tissue specificity can be obtained. In particular, a composition capable of improving the differentiation ability of pancreatic organoids can be obtained in the scaffold using the pancreatic dECM.

According to one embodiment of the present invention, a1) may be performed for 12 to 48 hours, a2) for 12 to 120 hours, a3) for 0.5 to 4 hours, a4) for 12 to 48 hours, a5) for 0.5 to 4 hours, and a6) for 2 to 12 hours. Specifically, a1) may be performed for 12 to 24 hours, a2) for 48 to 96 hours, a3) for 1 to 3 hours, a4) for 12 to 36 hours, a5) for 1 to 3 hours, a6) for 4 to 8 hours. When the steps are performed in the ranges described above, a composition for culturing organoids having excellent differentiation into tissue cells and having excellent tissue specificity can be obtained.

According to one embodiment of the present invention, the S2) lyophilizing is for lyophilizing a tissue decellularized in S1). The lyophilizing methods or specific conditions are not particularly limited, and various methods or conditions known in the art may be used. For example, the decellularized tissue may be frozen at -100°C to -50°C, and then dried at -70°C to -30°C for 1 to 10 days.

According to one embodiment of the present invention, the S3) pulverizing is for pulverizing the tissue lyophilized in S2). The pulverizing methods or sizes, and specific conditions are not particularly limited, and various methods or sizes and conditions known in the art may be used. For example, the lyophilized tissue may be put into a mortar and pulverized by using liquid nitrogen.

According to one embodiment of the present invention, the S4) lysing is for lysing the tissue pulverized in S3) above. In other words, the pulverized tissue can be lysed by adding a proteopeptic enzyme and an acid to the same, and stirring the resulting mixture.

Here, the present invention is characterized in that a proteopeptic enzyme and an acid are added to the pulverized tissue to lysate by stirring the resulting mixture at a speed of 330 rpm to 500 rpm for 72 to 96 hours. Preferably, the resulting mixture may be lysed by stirring at a speed of 330 rpm for 72 to 96 hours. In addition, the resulting mixture may be lysed by stirring at a speed of 330 rpm to 500 rpm for 96 hours. More preferably, the resulting mixture may be lysed by stirring at a speed of 330 rpm for 96 hours.

The inventors found that not in the process of decellularization but in the process of adding a proteopeptic enzyme and an acid to the decellularized, lyophilized and pulverized dECM and lysing the resulting mixture, the stirring time and speed are important to the physical properties and the subsequent culture effects of the decellularized tissue, and then completed the present invention.

As shown in the Examples to be described later, it was confirmed that when the decellularized tissue was lysed while stirring at a speed of 330 rpm to 500 rpm for 72 hours to 96 hours in the lysing, excellent rheological properties (physical properties) and culture effects were obtained, compared to the cases where the lysing was performed differently.

In other words, the modulus values representing the physical properties for each stirring time were found to be in the order of 24 h < 72 h ≒ 96 h > 120 h(24 h ≒ 120 h). In other words, it was confirmed that the physical properties were best when the stirring time was 72-96 hours. In addition, when the stirring time was 120 hours, the physical properties were so low that the organoid culture experiment itself was impossible, the culture tendency in the organoid culture was improved in the order of 24 h < 72 h < 96 h. Preferably, tissue properties were better when stirred for 72 hours, but organoid culture efficiency was better when stirred for 96 hours.

In addition, the modulus values representing the physical properties for each stirring speed were found to be in the order of 80 < 150 < 250 < 330 « 500 » 1000 (330 < 1000 « 500) rpm, and the best physical properties were found at 330-500 rpm. In addition, it was found that the culture efficiency in the organoid culture was in the order of 250 « 330 ≒ 500 » 1000 and that the dome formation was impossible at 80 rpm. Preferably, tissue properties were better when stirred at 500 rpm, but organoid culture efficiency was better when stirred at 330 rpm.

According to one embodiment of the present invention, the proteopeptic enzyme in S4) may be pepsin or trypsin. Preferably, it may be pepsin.

According to one embodiment of the present invention, the acid in S4) may be hydrochloric acid or acetic acid. Preferably, it may be acetic acid.

According to one embodiment of the present invention, the S5) adjusting the pH is for adjusting the pH by adding a basic solution to the lysate in S4) above. The method of adding a basic solution or the type of the basic solution to be used is not particularly limited, and various basic solutions known in the art may be used. The final pH can be adjusted to be 7 to 7.5.

Here, the present invention is particularly characterized in that the S5) adjusting the pH is performed within a range of 1°C to 10°C in order to prevent gelation. Preferably, it may be performed within a range of 2°C to 7°C. In other words, the pH adjusting process may not be performed at room temperature, but may be performed in a lower refrigeration temperature range.

In addition, the S5) adjusting the pH may be performed on ice in order to prevent gelation. The expression of performing "on ice" means that the lysate is present on the surface of the ice or provided in the ice. For example, the pH may be adjusted by adding a base, after inserting the container containing the lysate into ice.

The present inventors found that not in the process of decellularization but in the process of adjusting the pH by adding a basic solution to the decellularization lysate, the temperature (1°C to 10°C) and/or conditions (on ice) affect the hydrogelation of the composition and the subsequent encapsulation of organoids, and then completed the present invention.

As shown in Examples to be described later, when the process of adjusting pH by adding a basic solution to the decellularized lysate is performed "on ice (at 1°C to 10°C)," the gelation of the lysate may be prevented, and uniform hydrogelation may be possible. In addition, it was confirmed that the pancreatic dECM scaffold prepared in this way has excellent rheological properties (physical properties) and culture effects, compared to the case where the pancreatic dECM scaffold was prepared differently.

In other words, it was confirmed that the decellularization composition of which pH was adjusted on ice according to the present invention was able to form a uniform gel during the gelation process, but the decellularization composition of which pH was adjusted at room temperature (RT) underwent partial gelation and aggregation and so the dome shape was not maintained but broken and the physical properties thereof (complex modulus (G*)) were also low.

As described above, the present invention may be characterized in that the pH adjustment process is not performed at room temperature but in a lower refrigeration temperature range, and further, the basic solution is stored at a refrigeration temperature within a range of 1°C to 10°C in advance before use. A basic solution stored at a refrigeration temperature may preferably be used because the gelation prevention and uniform hydrogelation may be performed better.

According to one embodiment of the present invention, by the present invention described above, 95% or more of tissue cells, specifically 97% or more of the tissue cells can be removed, compared to the tissue cells before the treatment. In addition, the glucosaminoglycan content may be 90% to 120%, specifically 95% to 110%, compared to the glucosaminoglycan content before the treatment. In addition, the collagen content may be 200% to 2000%, specifically 500% to 1700%, compared to the collagen content before the treatment. Through this, an excellent cell removal rate may be obtained, and the useful components can be retained so that a scaffold based on the retained useful components may be used to obtain an organoid culture composition in which the organoid culture is performed well (culture, expansion, proliferation) and which has excellent differentiation into tissue cells and excellent tissue specificity.

According to one embodiment of the present invention, a cytokine-rich composition that is excellent for organoid culture may be obtained through the method described above. Specifically, compared to Matrigel, at least one cytokine selected from angiopoietin-1, insulin growth factor, TGF-beta, and placental growth factor may have been increased. Through this, an organoid culture composition in which the organoid culture is performed well (culture, expansion, proliferation) and which has excellent differentiation into tissue cells and excellent tissue specificity may be obtained.

The present invention also provides a composition for culturing pancreatic organoids prepared by the method described above.

According to one embodiment of the present invention, the composition may contain 0.1% to 10% (w/v) of dried decellularization tissue based on the total volume of the composition, specifically, 0.1% to 5% (w/v). In the case where the content described above is satisfied, when an organoid is cultured in the decellularization scaffold (dECM) based on the present invention, an excellent organoid culture composition in which the organoid culture is performed well (culture, expansion, proliferation) and in particular, which improves the organoid differentiation potency in the pancreatic dECM and well retains the mutation properties of a pancreatic cancer organoid may be obtained.

According to one embodiment of the present invention, the composition of the present invention may further include Matrigel.

The "Matrigel" is a protein complex extracted from EHS (Engelbreth-Holm-Swarm) mouse sarcoma cells (name of product manufactured by BD Bioscience) and may comprise an extracellular matrix, such as laminin, collagen and heparan sulfate proteoglycan, and fibroblast growth factor (FGF), epidermal growth factor (EGF), insulin-like growth factor (IGF), TGF-β or platelet-derived growth factor (PDGF).

According to one embodiment of the present invention, the composition may contain 0.1% to 10% (w/v) of dried decellularization tissue based on the total volume of the composition, specifically 0.1 to 50 (w/v). In the case where the content described above is satisfied, when an organoid is cultured in the decellularization scaffold (dECM) based on the present invention, an excellent organoid culture composition in which the organoid culture is performed well (culture, expansion, proliferation) and in particular, which improves the organoid differentiation potency in the dECM and well retains the mutation properties of a pancreatic cancer organoid may be obtained.

The present invention also provides a pancreatic organoid culture method comprising: culturing a pancreatic organoid in the composition for culturing pancreatic organoids described above.

The culture refers to a process of maintaining and growing cells under compatible conditions, and compatible conditions may mean, for example, the temperature at which the cells are maintained, nutrient availability, atmospheric CO₂ content, and cell density.

Compatible culture conditions for maintaining, proliferating, expanding and differentiating different types of cells are known and documented in the art. The compatible conditions for the formation of the organoid may be conditions that facilitate or allow cell differentiation and formation of multicellular structures.

The present invention also provides a pancreatic organoid culture method for culturing a pancreatic organoid, the method comprising: culturing a pancreatic organoid in the composition for culturing pancreatic organoids described above.

The present invention also provides an extracellular matrix scaffold for culturing organoids prepared from the composition for culturing pancreatic organoids described above.

The present invention also provides a pancreatic organoid culture method comprising: mixing the extracellular matrix support for culturing organoids and the pancreatic organoid.

The details of other Examples are included in the detailed description and drawings.

### ADVANTAGEOUS EFFECTS

The present invention is capable of creating an environment that is more similar to an actual tissue than a conventional Matrigel-based culture system, and in particular, exhibits an effect of facilitating tissue differentiation in pancreatic organoid culture and effectively developing into a form that is similar to an actual tissue.

In addition, when the decellularized tissue is stirred for a specific time and/or at a specific rpm in lysing the decellularized tissue with a proteopeptic enzyme and an acid according to the present invention, the physical properties of the decellularized tissue become excellent and the organoid culture rate is significantly increased.

In addition, when the process of adjusting the pH by adding a basic solution to the decellularization solution according to the present invention is performed under specific conditions, the physical properties of the decellularized tissue become excellent, gelation can be prevented, and uniform hydrogelation can be achieved.

### DESCRIPTION OF THE DRAWINGS

FIG. 1 shows a representative diagram of preparing a composition for culturing pancreatic organoids according to an embodiment of the present invention, and the organoid culture and application using the composition thereby.
FIG. 2 shows an analysis of dsDNA, GAGs, and collagen contents compared to the native tissues of pancreatic dECM according to an Example of the present invention.
FIGS. 3a and 3b show an analysis of the rheological properties of pancreatic dECM according to an Example of the present invention.
FIGS. 4a to 4e show an analysis of the remaining cytokines in the pancreatic dECM according to an Example of the present invention.
FIG. 5 shows an analysis of the histological characteristics of the pancreatic dECM according to an Example of the present invention.
FIG. 6 shows an analysis of the surface structure of the pancreatic dECM using FE-SEM according to an Example of the present invention.
FIG. 7 shows an analysis of the protein components of the pancreatic dECM using LC-MS/MS according to an Example of the present invention.
FIGS. 8a and 8b show an analysis of the gelation tendency (sol-gel transition) for selecting compatible culture conditions of pancreatic organoids according to an Example of the present invention. FIG. 8a shows the results obtained by preparing the pancreatic dECM according to an Example of the present invention at 1% and 1.5% and then analyzing the dECM alone and the mixtures prepared by mixing with Matrigel according to the presented ratios.
FIG. 8b shows an analysis of a frequency sweep analysis of scaffolds in conditions suitable for pancreatic organoid culture selected according to an embodiment of the present invention. FIG. 8b shows the results obtained by preparing the pancreatic dECM according to an Example of the present invention at 1% and 1.5% and then analyzing the dECM alone and the mixtures prepared by mixing with Matrigel according to the presented ratios.
FIG. 9 shows an analysis of cytotoxicity of pancreatic organoids using Live/Dead assay according to an embodiment of the present invention.
FIG. 10 shows the results of the evaluation of the proliferation of the pancreatic tumor organoids within the limits performed by using the CCK-8 analysis method according to an Example of the present invention. The analytical results are the proliferation tendencies obtained by culturing the mouse pancreatic normal organoids based on the pancreatic 1% dECM under the conditions of using the dECM alone and mixing with Matrigel according to the presented ratios.

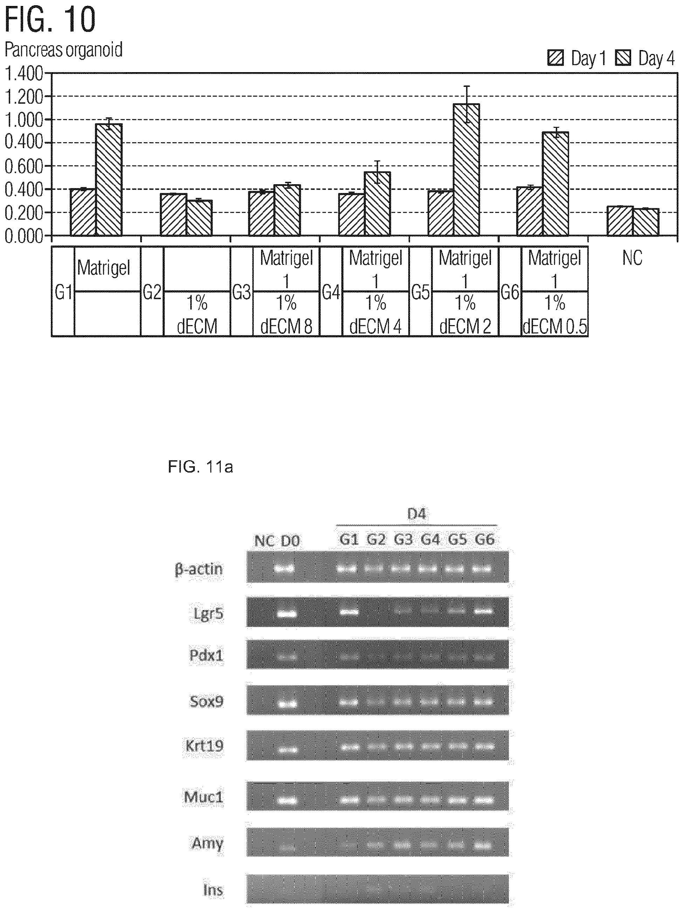

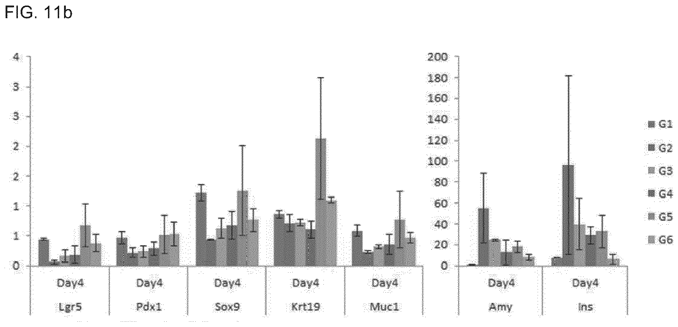

FIGS. 11a and 11b show verifying the culture effectiveness of the pancreatic organoids cultured in dECM through gene expression validation of mouse pancreatic normal organoids according to an embodiment of the present invention. FIG. 11a shows a qualitative analysis of pancreatic marker expression levels in mouse pancreatic normal organoids cultured on the basis of pancreatic dECM, and FIG. 11b shows the result of a quantitative analysis. β-actin: house keeping gene, Lgr5: Leucine-rich repeat containing G-protein coupled receptor 5, Pdx1: Pancreatic and duodenal homebox 1, Sox9: SRY-box containing gene 9, Krt19: Keratin 19, Muc1: Mucin 1, Amy : Amylase, Ins : Insulin. G1: Matrigel, G2: 1% dECM, G3: Matrigel 1 + 1% dCEM 8, G4: Matrigel 1 + 1% dCEM 4, G5: Matrigel 1 + 1% dCEM 2, G6: Matrigel 1+ 1% dCEM 0.5.

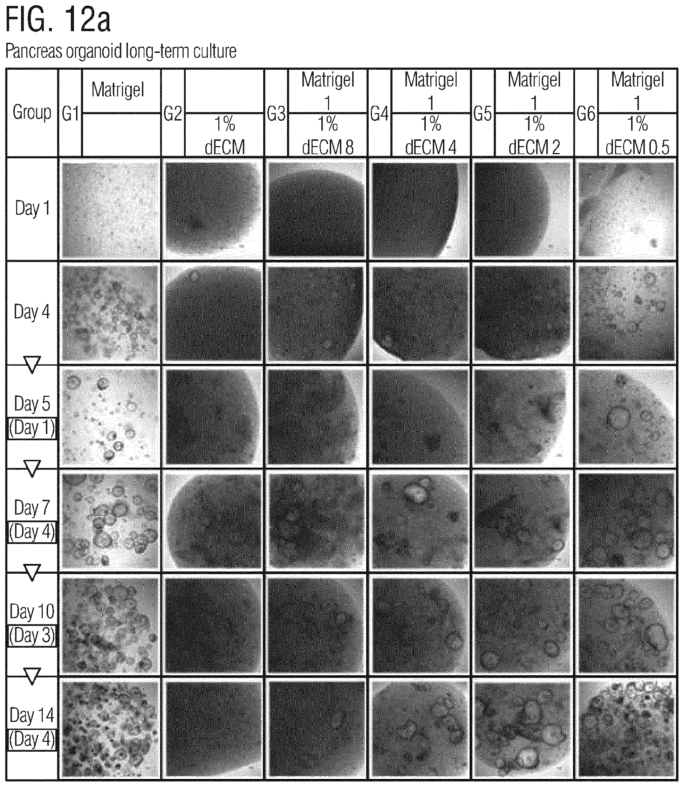

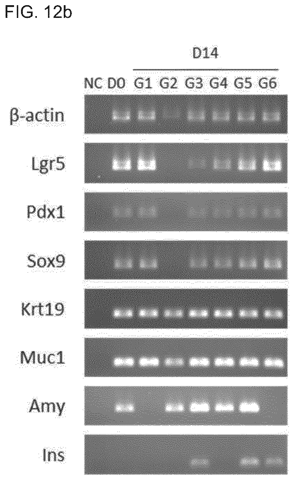

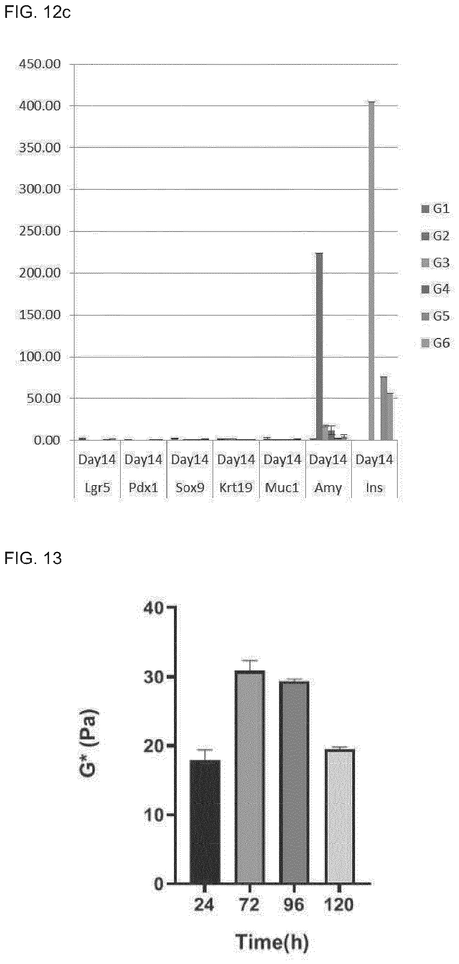

FIGS. 12a to 12c show the results of performing long-term culture of the mouse pancreatic organoids cultured in the dECM according to an Example of the present invention, confirming the organ-specific gene expression of the pancreatic organoids cultured in the dECM. FIG. 12a shows the results of microscopic observation of the long-term culture for 14 days of mouse pancreatic organoids that were cultured based on the pancreatic 1% dECM prepared according to an Example of the present invention and under the conditions of using the dECM alone and mixing with Matrigel according to the presented ratios, FIG. 12b shows a qualitative analysis of the level of pancreatic marker expression in mouse pancreatic organoids on the 14th day of culture, and FIG. 12c shows a quantitative analysis. G1: Matrigel, G2: 1% dECM, G3: Matrigel 1 + 1% dCEM 8, G4: Matrigel 1 + 1% dCEM 4, G5: Matrigel 1 + 1% dCEM 2, G6: Matrigel 1+ 1% dCEM 0.5.
FIG. 13 is a graph showing the modulus values of the pancreatic dECM when the stirring time was changed in the S4) lysing according to an Example of the present invention.
FIG. 14 is a graph showing the gelation kinetic value of the pancreatic dECM for each stirring time in the S4) lysing according to an Example of the present invention.
FIG. 15 is a photograph showing the state of culturing the pancreatic organoids for each stirring time in the S4) lysing according to an Example of the present invention.
FIG. 16 is a graph showing the proliferation rate of the pancreatic organoid culture for each stirring time in the S4) lysing according to an Example of the present invention.
FIG. 17 is a graph showing the modulus value of the pancreatic dECM when the stirring speed was changed in the S4) lysing according to an Example of the present invention.
FIG. 18 is a graph showing the gelation kinetic value of the pancreatic dECM at each stirring speed in the S4) lysing according to an Example of the present invention.
FIG. 19 is a photograph showing the culture state of the pancreatic organoids at each stirring speed in the S4) lysing according to an Example of the present invention.
FIG. 20 is a graph showing the proliferation rate (proliferation) of the pancreatic organoid culture according to the stirring speed in the S4) lysing according to an Example of the present invention.
FIG. 21 is a photograph for explaining the process of performing the S5) adjusting pH on ice according to an Example of the present invention.
FIGS. 22 to 24 are photographs showing the gelation state of the composition prepared by performing the S5) adjusting pH on ice according to an Example of the present invention.
FIG. 25 is a graph showing a modulus value of the composition prepared by performing the S5) adjusting pH on ice according to an Example of the present invention.
FIG. 26 is a graph showing a gelation kinetic value of the composition prepared by performing the S5) adjusting pH on ice according to an Example of the present invention.
FIG. 27 is a graph showing the change of the physical properties according to an increase of the temperature, when a pancreatic dECM + matrigel (2: 1) mixture was cultured using the composition prepared by performing the S5) adjusting pH on ice according to an Example of the present invention.

### BEST MODE

Since various transformations can be applied to the present invention, and the present invention can have various Examples, specific Examples are illustrated in the drawings and described in detail in the detailed description. However, this is not intended to limit the present invention to specific embodiments, and should be understood to include all transformations, equivalents, and substitutes included in the principles and scope of the present invention. In describing the present invention, if a specific description of a related known technology may obscure the gist of the present invention, the detailed description thereof will be omitted.

The terms used in the present application are only used to describe specific embodiments, and are not intended to limit the present invention. The singular expression includes the plural expression unless the context clearly dictates otherwise. In the present application, terms such as "comprise" or "have" are intended to designate that a feature, number, step, operation, component, part, or combination thereof described in the specification exists, but one or more other features It should be understood that this does not preclude the existence or addition of numbers, steps, operations, components, parts, or combinations thereof.

Terms such as first, second, etc. may be used to describe various features, but the features should not be limited by the terms. The above terms are used only for the purpose of distinguishing one feature from another feature.

### Example 1: Decellularization of Porcine Pancreatics

### (1) Removing of non-pancreatic tissue and slicing of pancreatic tissue

After washing the pancreatic tissue in running water and removing the non-pancreatic tissue with sterilized operating scissors, the pancreas is transferred to a plastic bag using forceps and then frozen at -80°C for 1 hour. After cutting the frozen pancreatic tissue to a thickness of 1 mm, it is transferred to a 500 mL plastic container. For the container used at this time, it is recommended to use a plastic container with a lid to prevent tissue contamination and evaporation of the solution.

### (2) Decellularization reagent treatment

Before the reagent treatment, the tissue is washed with distilled water (dH₂O; secondary water) within 12 hours. After washing, the tissue is treated with 1% Triton-X 100 within 84 hours. In this step, the amount of the tissue is reduced. The tissue is treated with isopropanol (IPA) for 2 hours to remove residual adipose tissue. In this step, the tissue becomes tough. After removing IPA, the tissue is treated with 1X PBS (phosphate buffered saline) for 24 hours. For sterilization, the tissue is treated with ethanol containing peracetic acid for 2 hours. Hereinafter, tertiary distilled water (ddH₂O) is used as all base buffers. The tissue is performed with 1X PBS for 6 hours to remove residual reagents. The tissue is moved to a 50 mL conical tube by using forceps. After freezing the tissue at -80°C, the tissue is lyophilized at -50°C for 4 days.

In the entire decellularization process, the washing process employs a stirrer at a speed of 100-150 rpm. In each of the other reagent processing steps, the stirring speed is kept within 100 rpm to ensure that the tissue and the reagent can react sufficiently. In all steps, forceps and scissors are used to loosen the tissue in order to prevent congelation of the tissue. In addition, the beaker and the magnetic bar are washed with distilled water in each step in order to remove the remaining reagent. In the entire decellularization process, a digital orbital shaker is used at 100-200 rpm at 4° C., and forceps are used to loosen the tissue in order to prevent congelation of the tissue. The plastic container are washed with distilled water in each step in order to remove the remaining reagent.

### Example 2: Preparation of Pancreatic dECM Scaffolds

The lyophilized pancreatic dECM prepared in Example 1 is pulverized with liquid nitrogen by using a mortar and a pestle. The pancreatic dECM is put into a 50 mL conical tube and lysed in 0.5 M acetic acid with pepsin for 96 hours. A cell strainer is used to filter the pancreatic dECM powder that is not lysed. In order to prevent gelation, the steps hereinafter should be performed on ice. After adding cold 10X PBS before treating with 10 N sodium hydroxide (NaOH), the pancreatic dECM is homogeneously mixed by using a vortex mixer. A pH indicator strip is used to observe the change of pH, and 10 N sodium hydroxide (NaOH) is added to adjust the final pH to 7-7.5. Cold 1X PBS is added to adjust to the final volume.

### Example 3: Pancreatic dECM Biochemical Assay

As the verification of the decellularized pig pancreatic tissue, the contents of dsDNA, GAGs and collagen in the decellularized tissue is confirmed, compared to the native tissue.

### (1) Confirmation of dsDNA content in decellularized pancreatic tissue

GeneJET Genomic DNA Purification Kit was used to separate dsDNA from the native and decellularized tissues. The separation was carried out in the same manner as described in the manufacturer's protocol. The dsDNA content in the decellularized tissue compared to the native tissue was confirmed by using Quant-iT^{™} PicoGreen^{™} dsDNA Assay Kit. The assay was carried out in the same manner as described in the manufacturer's protocol.

### (2) Preparation of Solution for Confirming the Content of Glucosaminoglycans (GAGs) and Collagen in Decellularized Pancreatic Tissue

A papain solution is prepared for the digestion of dECM. In the preparation of all solutions (papain, buffer, etc.), the amount of the solution can be adjusted according to the number of samples. The specific process is described below. (1) 0.1 M sodium phosphate (monobasic), 0.5 mM Na2-EDTA, and 5 mM cysteine-hydrochloric acid are dissolved in autoclaved distilled water. (2) The pH of the solution is adjusted to 6.5 by adding 10 N sodium hydroxide. (3) A papain stock prepared at 10 mg/mL is added to the solution above, and the resulting mixture is mixed well with a vortex mixer to be mixed homogeneously.

A solution for the dimethyl-methylene blue (DMMB) assay is prepared. The specific process is described below. (1) To prepare a DMMB dye, 1,9-dimethyl-methylene blue zinc chloride double salt, glycine, and sodium chloride are dissolved in autoclaved distilled water. The pH is adjusted to 3 by adding 0.5 M HCl solution, while measuring the change of pH by using a bench-top pH meter. Then, after filtering by using a filter, an aliquot of the solution is taken in a conical tube, which is wrapped with aluminum foil and stored. ② A 10 mg/mL chondroitin sulfate A solution stock is prepared at 100 µg/mL by using 1X PBS.

A solution for the hydroxyproline assay is prepared. The specific process is described below.
① Chloramine Working Solution: Sodium acetate, citric acid, and sodium hydroxide are dissolved in distilled water, and glacial acetic acid, toluene, and IPA are added. The chloramine working solution is stored at 4°C and can be used for about 6 months.
② Chloramine T solution: After dissolving Chloramine T in the chloramine working solution, IPA is added. The mixture is mixed well with a vortex mixer to be mixed homogeneously. However, the Chloramine T solution should be prepared immediately before use.
(3) P-DAB solution: P-DAB is added to perchloric acid, IPA is added, and the mixture is mixed well. However, the P-DAB solution should also be prepared just before use and since it is sensitive to light, the tube should be wrapped with aluminum foil until use.

### (3) dECM Digestion of Decellularized Pancreatic Tissue

The lyophilized tissue is put into a 1.7 mL microcentrifuge tube (e-tube) to which the papain solution is added, and the resulting mixture is mixed well with a vortex mixer. The e-tube containing the tissue is inserted into rubber racks, which are floated on water added into a beaker, and then the tissue is digested at 60°C for 16 hours. However, the tissue should be completely lysed without any remaining tissue. Centrifugation is performed at 12000 x g for 20 minutes, and the supernatant is taken and transferred to a new e-tube.

### (4) Confirmation of the content of Glycosaminoglycans (GAGs) and Collagen in Decellularized Pancreatic Tissue

A DMMB assay is performed to quantify the GAGs in the dECM. The specific process is described below. ① A standard in a specific range according to the purpose of the experiment is prepared by using chondroitin sulfate A solution stock prepared at 100 ug/mL and distilled water. ② The standard and samples are loaded in triplicate into a 96-well plate at 40 µL/well. ③ The DMMB dye is added at 160 µL/well by using a multi-channel pipettor. ④ The absorbance is measured at a 525 nm wavelength with a microplate reader.

A hydroxyproline assay is performed to quantify the amount of collagen in the dECM. The specific process is described below. ① The tissue lysed in the papain solution and the same amount of hydrochloric acid are subject to a reaction in an oven at 120°C for 16 hours. ② After the reaction, the residue remaining on the glass wall is dried and cooled at room temperature for 3 hours, and then lysed again in 1X PBS. (3) The completely lysed sample is transferred to an e-tube and centrifuged at 5,000 x g for 10 minutes at 4°C, and only the supernatant is transferred to a new e-tube. ④ During the centrifugation, the 100 ug/mL hydroxyproline solution stock is diluted with distilled water to prepare a standard in a specific range according to the purpose of the experiment. (5) The standard and samples are loaded in triplicate into a 96-well plate at 50 µL/well. ⑥ The chloramine T solution in a volume of 50 µL is added, and then the resulting mixture is subject to a reaction at room temperature for 20 minutes. ⑦ The P-DAB solution in a volume of 50 µL is added and then the resulting mixture is subject to a reaction 60°C for 30 minutes. Since P-DAB is sensitive to light, the loading should be conducted with the light off, and then the plate should be wrapped with aluminum foil to perform incubation. (8) After cooling for 30 minutes at room temperature, the absorbance is measured at a 540 nm with a microplate reader.

### (5) Experiment results

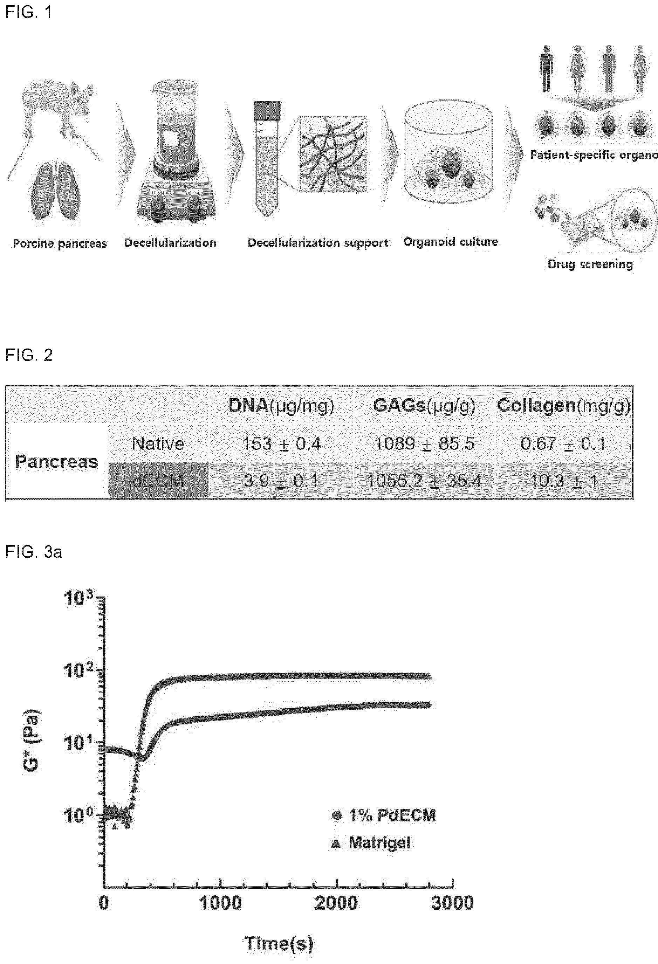

The specific experimental results are shown in FIG. 2. In the case of the pancreatic dECM, compared to the native group, the DNA content was 2.48% and 2.05% respectively, and more than 97% of the cells were removed. In addition, the content of GAGs, of which loss occurs mainly in the decellularization process, was about 96% in the pancreatic dECM, which was similar to that of the native group. Therefore, the decellularization process was considered as having been well established. The collagen content in the pancreatic dECM was 1537% compared to the native group, and so it was verified that cells were removed during the decellularization process and the main components of the extracellular matrix were well preserved.

### Example 4: Rheological Characterization of Pancreatic dECM

### (1) Experimental Method

The pancreatic dECM scaffold is prepared at pH 7 to evaluate the rheological properties. The 20 mm cone plate geometry (cone diameter of 20 mm at a 2° angle) is set in the rate-controlled mode of a rheometer. An experiment sequence in the installed software program (TRIOS) is crated to measure the viscosity, gelation kinetics, and dynamic modulus of the dECM scaffold.
*Gelation kinetics: The dECM scaffold is placed on a plate, and the complex modulus (G*) is calculated by measuring the storage & loss modulus of the dECM scaffold at 4-37°C at an incremental increase rate (time-sweep mode) of 5°C/min.
*Dynamic modulus: Before the measurement, the dECM scaffold is placed on the plate at 37°C for 30 minutes. The frequency-dependent storage modulus (G') and loss modulus (G'') of the dECM scaffold are measured at 2% strain within a range of 0.1-100 rad/s.

The amount of the dECM loading is set to be 250 µL, and the running GAP is set to be 500 um. Before running, the dECM that has escaped from the periphery of the cone plate is removed as much as possible with a thin scraper or the like.

### (2) Experimental Results

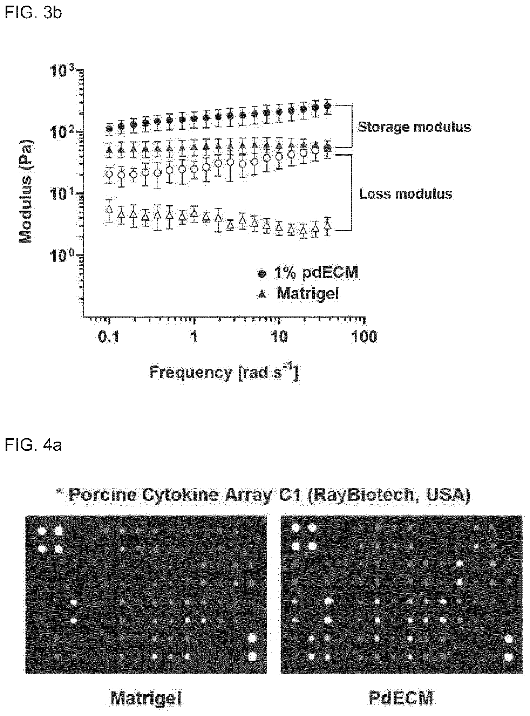

The analytical results are shown in FIGS. 3a and 3b. Matrigel and the neutralized pancreatic dECM hydrogels exhibit thermal response behavior, show a solution-like phase at a temperature below 15°C, and transform into a gel-like phase (crosslinked gel) when the temperature is increased to a physiological temperature (37°C). The analysis of the rheological properties of Matrigel and the pancreatic dECM, performed by using a rheometer, showed that stable hydrogels were formed in all groups. It was confirmed that the gelation of pancreatic dECM occurred slowly but formed a higher complex modulus. Finally, the neutralized pancreatic dECM showed a fluid-to-gel transition for 50 minutes (FIG. 3a). In addition, it was confirmed that the cross-linked pancreatic dECM hydrogel, after the gelation at a physiological temperature (37°C), exhibited a very stable crosslinking process under dynamic external forces in a range from a low shear frequency to a high shear frequency (0.1-100 rad/s) (FIG. 3b).

### Example 5: Surface Characterization of Pancreatic dECM Using FE-SEM

The pancreatic dECM scaffold is adjusted to pH 7.

### (1) Freeze Dry Pretreatment (Application of pancreatic dECM)

The pancreatic dECM gelated at 37°C for 30 minutes is immersed in 4% paraformaldehyde to fix. Samples are washed 3 times with 1X PBS for 10 minutes each. Ethanol at 30%, 50%, 70%, 90% and 100% is prepared and treated according to the sequence for 10 minutes each. After rapidly cooling with liquid nitrogen (LN), lyophilize. This process is for preserving the internal structure, and if this process is omitted and freeze-dried, structural damage may occur. After coating with platinum (Pt), imaging is performed.

### (3) Experimental results

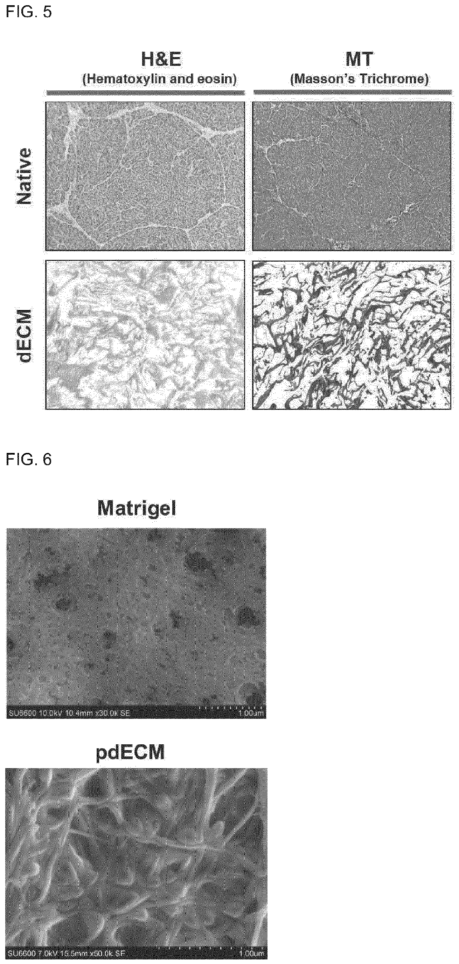

The results are shown in FIG. 6, and it was confirmed that the ECM components were well formed in the form of fiber.

### Example 6: Analysis of Residual Cytokines in Pancreatic dECM

### (1) Solution preparation and pancreatic dECM scaffold preparation

### (2) Pancreatic dECM protein quantification

The pancreatic dECM scaffold is prepared by adjusting the pH to 7. After adding the RIPA buffer to the neutralized dECM, which is kept in ice and subject to a reaction for about 30 minutes. The mixture is mixed well by using a vortex mixer once every 5 minutes. The total protein in the dECM is quantified by using the BCA protein assay kit. The standard is prepared by diluting a 2 mg/mL stock of bovine serum albumin (BSA) with ddH₂O. The pancreatic dECM dissociated in the RIPA buffer is used as a stock solution, and samples are prepared by diluting by factors of 2/5/10/50/100/200/500/1000 times with ddH₂O. The standard and the samples are loaded in triplicate into a 96-well plate at 20 µL/well each. The blank is the RIPA buffer. The required amount of the BCA solution is calculated, and Solution A and Solution B are mixed at the ratio of 49:1. It should be ensured that the tube is wrapped with aluminum foil, and Solution A and Solution B solutions are not mixed in advance but are mixed after loading the samples. The mixed solution is poured into the reservoir and loaded at 160 µL/well each by using a multi-channel pipettor. The entire 96-well plate is wrapped with aluminum foil and subject to a reaction in an incubator at 37°C for 30 minutes. The absorbance is measured at a 540 nm wavelength with a microplate reader. A standard curve is drawn and the total protein in the samples is calculated. The dilution factor was determined within the recommended protein loading range (load 50 to 500 µg of total protein) provided in the cytokine array protocol. The diluted solution was prepared by diluting the pancreatic dECM stock solution to a concentration of 50 µg/mL so that the total protein became 50 µg. The analysis method was carried out in the same manner as the manufacturer's protocol (RayBio^{®} C-Series Porcine cytokine array 1).

### (3) Experimental results

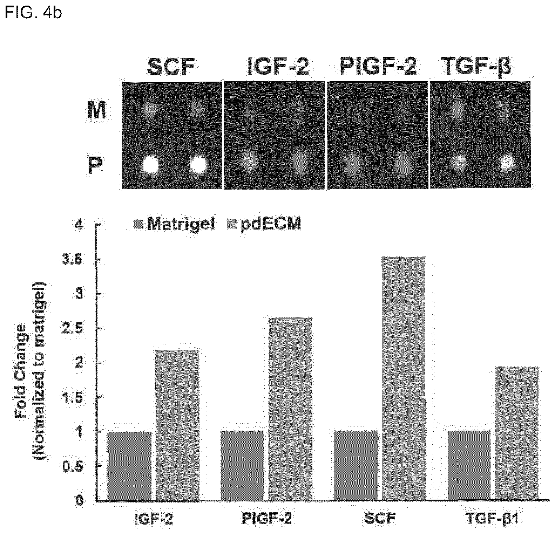

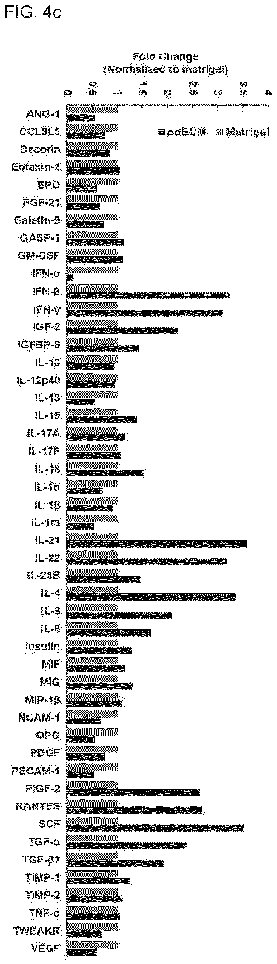

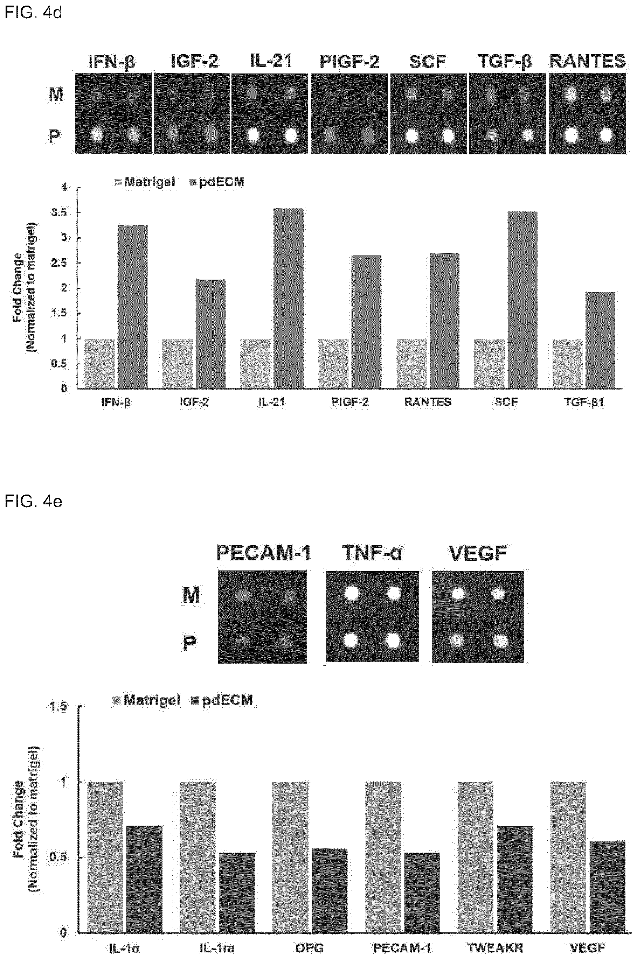

The results are shown in FIGS. 4a to 4e, and FIGS. 4a to 4e show the results of analyzing the residual cytokines in the pancreatic dECM. Among them, it was confirmed that stem cell growth factor (SCF) was present in the pancreatic dECM more than twice as high as Matrigel, in addition, major factors affecting organoid culture, such as insulin-like growth factor (IGF), TGF-β, or placental growth factor (PIGF), are contained in a large amount, compared to Matrigel. Therefore, the pancreatic dECM contains a large amount of cytokines produced and secreted by immune cells, vascular cells, and other stromal cells in actual tissues, and thus can mimic the tissue microenvironment more similarly. In particular, in addition to the provision of extracellular matrix, the pancreatic dECM contains cytokines related to angiogenesis and cell proliferation in a large amount, and thus is more effective in the organoid culture than Matrigel.

### Example 7: Evaluation of Histological Characteristics of Pancreatic dECM

Tissue staining (Hematoxylin & Eosin, Masson's trichrome stain) of the pancreatic dECM prepared in Examples 1 and 2 was performed.

As shown in FIG. 5, the histological (H&E, MT staining) analysis confirmed that the cells were removed from the tissue after the decellularization process, while the extracellular matrix component mimicking the biochemical microenvironment was maintained.

### Example 8: Analysis of residual protein in pancreatic dECM

The residual protein of the pancreatic dECM prepared in Example was analyzed by using LC-MS/MS (Liquid Chromatography-Mass spectrometry).

### (1) Sample pretreatment

*Protein unfolding: In order to split the remaining protein into peptide fragments, it is first lysed in a buffer to break the covalent bond.
* Deglycosylation: This is a process that is specifically applied to glycoproteins. Since glycoproteins have sugar chains in the middle of the sequence and the sugar chains may cause problems in the mass value later, the sugar portions are removed and the glycoprotein portions are changed into the forms of normal peptides.
*Digestion : Proteins are decomposed with LysC/Trypsin, etc.
*Clean up : Detergents such as SDS and salt components in the buffer are removed. This process must be taken for accurate detection of peptide ions because salt interference can be problematic.

### (2) Analysis using LC-MS/MS

The database of the individuals is imported and analyzed by using the sequence library.

### (3) Experimental results

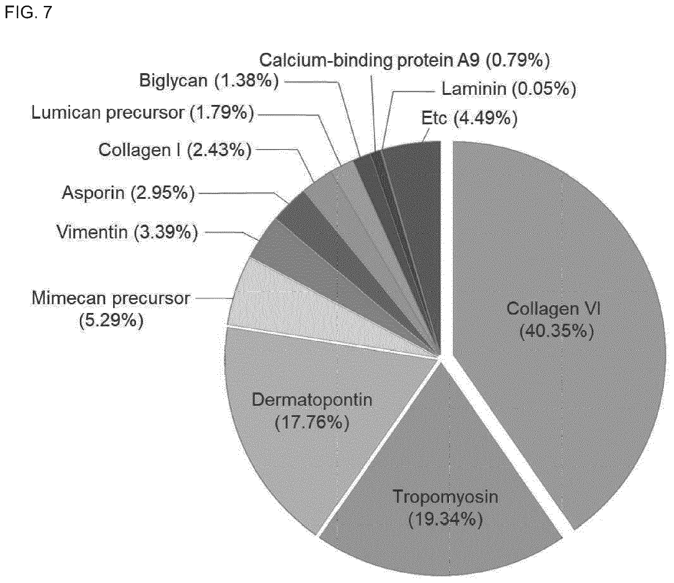

As shown FIG. 7, the experimental results confirmed that the pancreatic dECM contained a large amount of collagen VI, which is related to cell survival and functional improvement.

These proteins are known to be related to the survival and function of the islet responsible for secretion of insulin in the pancreas, therefore, when pancreatic organoids are cultured in a support mixed with pancreatic dECM alone or Matrigel developed in the present invention, it can help improve growth and differentiation of the organoid. Additionally, the results confirmed that tissue-specific extracellular matrix proteins remained even after the decellularization process so that the decellularization-based pancreatic dECM scaffold developed in the present invention can help the organoid growth and improve the organoid differentiation and functions.

### Example 9: Selection of Formulations Compatible for Organoid Culture

To perform organoid culture with the pancreatic dECM, which had been completely prepared for the use as a support, hydrogel formulation conditions similar to the gelation tendency of a scaffold (Matrigel) that is often used for organoid culture were screened. The verification was performed by using the dECM alone or by using a mixture of Matrigel and the dECM.

### (1) Selection of Formulations Compatible with Pancreatic dECM Organoid Culture

*The pancreatic dECM was tested based on the concentrations of 1%, and 1.5%, the dECM at each concentration was used alone or mixed with Matrigel (dECM:Matrigel ratio = 2:1, 1:1, 1:2) for comparison. The dECM at each concentration was used alone or mixed with Matrigel (dECM:Matrigel ratio = 8:1, 4:1, 2:1, 1:1, 1:2) for comparison. The overall details are similar to "Example 4: Rheological Characterization of Pancreatic dECM".

### (2) Gelation Tendency (sol-gel transition) and Frequency Sweep Analysis of Scaffold

To evaluate the rheological properties, the pancreatic dECM scaffold is prepared by adjusting the pH thereof to 7-7.5. The measuring plate PP25 is mounted on the rheometer to perform the evaluation. The amount of dECM loading is 300 µL or more, and the running GAP is set to 500 um for the pancreatic. Before running, the dECM that has escaped from the periphery of the measuring plate is removed as much as possible with a scraper. The sol-gel transition, complex viscosity, and frequency sweep of the dECM scaffold are measured by creating an experiment under the following conditions in the installed software program (RheoCompass 1.19).
*Gelation kinetics: The dECM support is placed on a plate, and the storage & loss modulus of the dECM support is measured under the conditions of 2% strain, 100 rad/s, and 2°C/min at 4-37°C for 30 minutes to calculate the sol-gel transition tendency and complex viscosity (η*).
*Frequency sweep: The frequency-dependent storage modulus (G') and loss modulus (G") of the completely gelated dECM scaffold is measured by changing the frequency from 500 rad/s to 1 rad/s at 2% strain and at 37°C.

### (3) Experimental Results

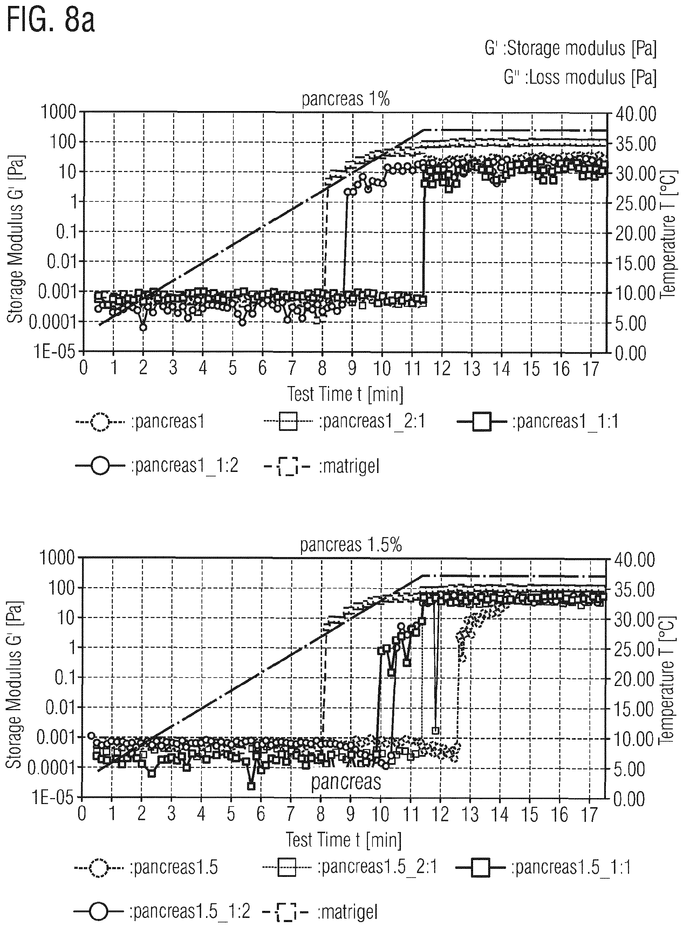

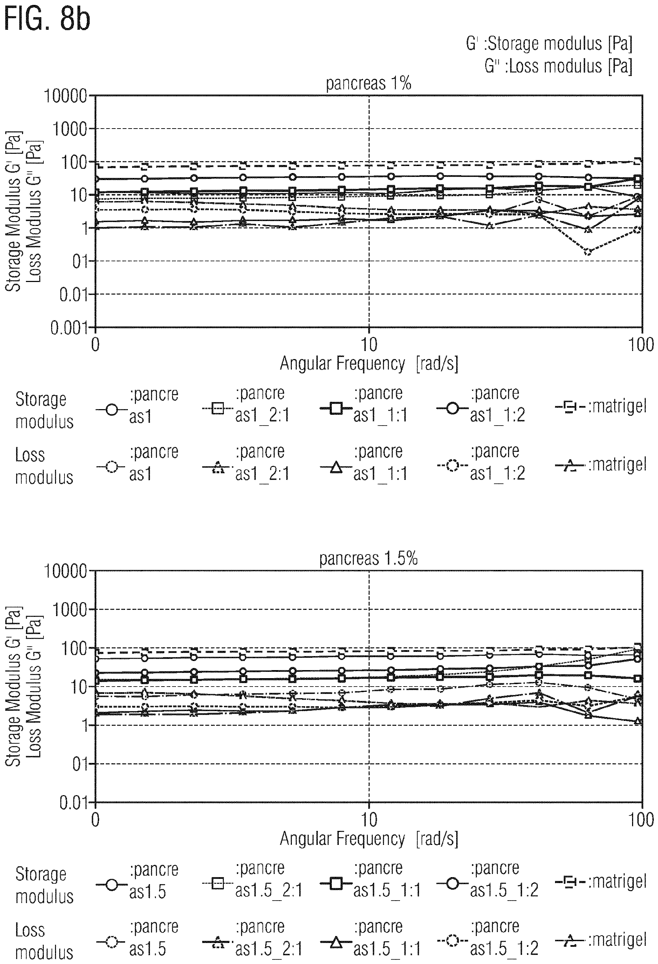

The results are shown in FIG. 8a and FIG. 8b. As in Example 5, Matrigel and neutralized pancreatic dECM hydrogels exhibit thermal reaction behavior and a solution-like phase at a temperature of lower than 5°C. As the temperature increases to 37°C, the same as in the body, it is transformed into a gel-like state. The sol-gel transition and frequency sweep were analyzed by a rheometer with Matrigel as a control, with the pancreatic dECM alone, and with the mixed gels prepared by mixing the same with Matrigel at the ratios described in Example 9 (1), and the results observed from each organ are described below.

FIGS. 8a and 8b show the results of analyzing the dECM alone and the mixtures prepared by mixing the same with Matrigel at the ratios described above, after preparing the pancreatic dECM at 1% and 1.5% according to one embodiment. In both the control group (Matrigel) and the presented experimental group, gelation similar to that of Matrigel was observed, and among pancreatic 1% and 1.5% dECM, and mixtures mixed with Matrigel based on the dECM, as the ratio of pancreatic dECM increased, it was observed that gelation progressed more slowly than Matrigel. According to analytical results of the viscoelasticity (frequency sweep) of the hydrogel of the pancreatic dECM gelated at 37°C and mixtures thereof, matrigel-like physical properties were observed in pancreas 1% and 1.5% dECM 1% and 1.5% dECM and their mixtures, as the mixing ratio of Pancreas 1% dECM increased, a tendency to have lower physical properties than Matrigel was confirmed, Pancreas 1.5% dECM showed the most similar physical properties to Matrigel when used alone. A suitable formulation for organoid culture based on neutralized pancreatic dECM is based on pancreatic 1% dECM according to the results of Examples 10 and 11 and FIG. 8a.

Finally, the neutralized pancreatic dECM and the mixture prepared at the selected ratio based on the results described above among the mixtures described in Example 9 were subject to gelation for 30 minutes (FIGS. 8a, 8b). In Example 10, in consideration of gelation stabilization, gelation was performed at 37°C for 40 minutes or longer.

### *Example 10: dECM-Based Organoid Culture

To evaluate the organoid culture compatibility of the pancreatic dECM that has completely been prepared for use as a scaffold, organoid culture is performed based on the dECM..

### (1) Mouse Pancreatic Organoid Culture and dECM Embedding

To culture a mouse pancreatic organoid, based on Advanced DMEM/F12, a medium is prepared by mixing Glutamax, HEPES, and Anti-anti. For the pancreatic organoid culture, growth factors such as N2, B27, N-acetylcysteine, mEGF, hNoggin, Rspondin-1, Nicotinamide, hGastrin, and hFGF10 are added. To culture in the dECM the pancreatic organoid that is being cultured, the organoid that is being cultured in Matrigel is recovered.

The specific process is described below. ① After removing the old medium, adding PBS. ② The Matrigel in which the pancreatic organoid is growing is physically destroyed through pipetting. ③ The organoid is disassembled with a syringe and a needle. ④ Pipetting is additionally performed by using a 10 ml tip. ⑤ Centrifugation is performed at 4°C and 200xg for 5 minutes. ⑥ The supernatant is removed to obtain an organoid pellet. ⑦ The amount required for embedding is calculated and the organoid pellet is mixed with the dECM scaffold made in Example 3 (a mixture prepared according to the mixing ratio of Matrigel and dECM). ⑧ The dECM mixture of 40-50 ul is dropped into a 24-well plate (or culture plate). (9) Incubation is performed at 37°C for 30-40 minutes. ⑩ The organoid culture medium containing Y-27632 is added. ⑪ The medium is replaced by an organoid culture medium that does not contain Y-27632 once every 2-3 days. ⑫ After 7-14 days following the subculture, depending on the state of the organoid, subculture is performed for long-term culture (14 days).

### Example 11: Cytotoxicity Evaluation of dECM-Based Organoids

To evaluate the culture compatibility of the organoid cultured on the dECM scaffold, live cells are confirmed by a Live/Dead assay to confirm that the dECM scaffold is nontoxic to the organoid culture.

### (1) Live/Dead Assay of Organoid in dECM-based Culture

The toxicity of the dECM scaffold was confirmed by staining and observing the live cells and dead cells of the organoid cultured in the dECM by using the Invitrogen Live/Dead^{®} Viability/Cytotoxicity Kit. The dECM scaffold in which organoid is being cultured is washed with pre-warmed DPBS (1X) 3 times for at 37°C for 5 minutes each. Afterwards, the method was performed in the same manner as the manufacturer's protocol.

### (2) Experimental results

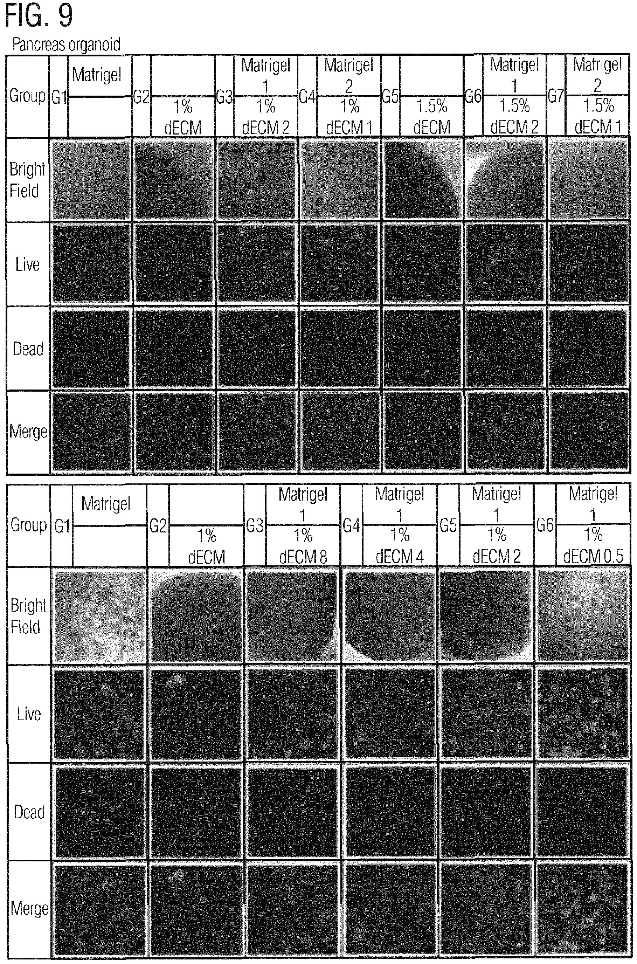

As shown in FIG. 9, the experimental results confirmed that there is no cytotoxicity in culturing organoids in the pancreatic dECM prepared according to an Example of the present invention and in the organoid culture-compatible hydrogel selected in Example 9.

As mentioned in Example 9, when mouse pancreatic organoids were cultured based on pancreatic 1% dECM and 1.5% dECM, it was confirmed that the organoids were effectively cultured in pancreatic 1% dECM (FIG. 9 first picture), based on the results, as a result of experiments with varying mixing ratios of pancreatic 1% dECM and Matrigel,

It was observed that in the experimental group in which pancreatic 1% dECM and Matrigel were mixed at 8:1, 4:1, 2:1, and 0.5:1, the culture was similar to Matrigel without toxicity (second figure in FIG. 9).

### Example 12: Evaluation of Proliferation of dECM-Based Organoids

To evaluate the culture compatibility of the organoid cultured on the dECM scaffold, the proliferation potential of the organoid in the dECM is evaluated with the CCK-8 assay, which confirms whether the organoid cultured on the dECM scaffold proliferates based on the respiration rate of the cells.

### (1) dECM-Based CCK-8 Assay of Organoid in Culture

The proliferation of an organoid (and cell) being cultured in the dECM was confirmed by measuring the respiration volume of the organoid cultured in the dECM by using DOJINDO's CCK-8 assay kit. The dECM scaffold in which the organoid is being cultured is washed with pre-warmed DPBS (1X) 3 times at 37°C for 5 minutes each. Afterwards, the method was performed in the same manner as the manufacturer's protocol. In the analysis of the results, the occurrence of the proliferation was confirmed by comparing the absorbance values of the samples for each period.

### (2) Experimental Results

The results are shown in FIG. 10, confirming the cell proliferation tendency when culturing the organoid based on the pancreatic dECM. N.C, referring to a negative control value, was measured in a blank in the absence of cells.

The results showed that in the case of culturing a mouse pancreatic normal organoid based on the pancreatic dECM, when cultured with pancreatic 1% dECM alone, the number of cells did not proliferate during 4 days of culture, it was confirmed that cell proliferation occurred in all experimental groups in which pancreatic 1% dECM and Matrigel were mixed at the ratio of 8:1, 4:1, 2:1, and 0.5:1. Among them, when pancreatic 1% dECM and Matrigel were mixed at a ratio of 2:1, it was observed that the number of cells constituting organoids proliferated similarly or superiorly to Matrigel.

Finally, it was confirmed that the hydrogel mixed with pancreatic 1% dECM and Matrigel at a ratio of 2:1 had a similar or superior effect on organoid growth than Matrigel.

### Example 13: Validation of Culture Effectiveness of dECM-Based Human-Derived Pancreatic Tumor Organoid

To evaluate the culture compatibility of the organoid cultured on the dECM scaffold, it is confirmed whether the organoid cultured on the dECM scaffold is effective with the tumor organoid cultured on the scaffold which is often used in the organoid culture (Matrigel). By comparing gene expression between mouse pancreatic organoids cultured on the dECM scaffold and mouse pancreatic organoids cultured in the control group, the expression of genes representing the characteristics of each organ in dECM was confirmed.

### (1) RNA extraction from mouse pancreatic organoids cultured in pancreatic dECM

Using TRIzol^{™} reagent, mouse pancreatic organoids cultured on pancreatic dECM and Matrigel (control) were sampled. (①~⑧ Process) RNA was extracted using Qiagen RNeasy mini kit. It proceeded in the same way as the manufacturer's protocol. (After ⑨) The specific process is as follows.

① Remove the medium of mouse pancreatic organoids cultured in Matrigel and dECM. (2) Add 1ml of TRIzol^{™} reagent, crush it physically by pipetting, and transfer it to a microcentrifuge tube. (3) React at room temperature for 5 minutes. (4) Add 200ul of chloroform and mix well by vortexing. (5) React at room temperature for 3 minutes. (6) Perform centrifugation at 4°C, 12,000xg, 15 minutes. ⑦ Transfer the supernatant to a new microcentrifuge tube (about 500ul) ⑧ Add 0.5 volume of 100% ethanol to the supernatant in ⑦ and mix well. ⑨ Put the mixed sample into the RNeasy mini spin column. The subsequent process proceeds according to the Qiagen RNeasy kit protocol.

### (2) cDNA synthesis based on extracted RNA

Using the Applied biosystems High-Capacity cDNA Reverse Transcription Kit with RNase Inhibitor, cDNA was synthesized based on the RNA extracted in (1). It proceeded in the same way as the manufacturer's protocol.

### (3) RT-qPCR using the synthesized cDNA as a template

Real time qPCR was performed using the cDNA synthesized in (2) as a template using Applied biosystems PowerUp SYBR Green Master Mix. In order to confirm the culture effectiveness of normal mouse pancreatic organoids, gene expression levels were confirmed with the following markers: Pancreatic progenitor (Pdx1), Ductal lineage (Sox9, Krt19), Endocrine (Muc1, Insulin), Acinar cells (Amylase), Lgr5+ stem cell (Lgr5), Housekeeping (β-actin). The result analysis was analyzed as a band appearing through electrophoresis.

In addition, in comparison to the level of each gene expression on Day 0, the level of gene expression was compared and analyzed according to the presence and absence of dECM.

### (4) Experimental Results

Experimental results are shown in FIGS. 11a and 11b. FIG. 11a shows a result of confirming pancreatic organoid markers of mouse pancreatic organoids cultured in a pancreatic dECM-based hydrogel. Among them, the Lgr5 marker, which can determine the expression level of Lgr+5 stem cells, which is known to be key in organoids, was expressed in all except when pancreatic 1% dECM was used alone, In the hydrogel mixed with pancreatic 1% dECM and Matrigel, it was observed that the expression was similar to that of Matrigel as the content of Matrigel increased. In addition, it was confirmed that bile duct markers Sox9 and Krt19, and endocrine cell marker Muc1 were expressed in all experimental groups, and Pdx1 expressed in pancreatic progenitor cells was also expressed in all experimental groups. In addition, it was confirmed that amylase (Amy) expressed in acinar cells was also expressed in all experimental groups. In particular, insulin (Insulin ; Ins) found in pancreatic beta cells was confirmed to be expressed when pancreatic 1% dECM was used alone or in combination with Matrigel, when the content of dECM was high.

Therefore, when organoids are cultured by mixing pancreatic 1% dECM with Matrigel, organoids similar to those when cultured on Matrigel can be cultured, In particular, by increasing the expression level of pancreatic-specific markers, it can be effective for pancreatic organoid culture and differentiation.

In addition, organ-specific gene expression of pancreatic organoids cultured in dECM was verified by long-term culture of mouse pancreatic organoids according to an embodiment of the present invention. Accordingly, FIG. 12a is a result of microscopic observation about long-term culture of mouse pancreatic organoids for 14 days under conditions in which single dECM or single dECM and Matrigel were mixed in the presented ratio based on pancreatic 1% dECM of the present invention, FIG. 12b is a qualitative analysis of the pancreatic marker expression levels of mouse pancreatic organoids on day 14 of culture, and FIG. 12c is a quantitative analysis. G1: Matrigel, G2: 1% dECM, G3: Matrigel 1 + 1% dCEM 8, G4: Matrigel 1 + 1% dCEM 4, G5: Matrigel 1 + 1% dCEM 2, G6: Matrigel 1+ 1% dCEM 0.5.

As a result of the experiment, when organoids were cultured by mixing pancreatic 1% dECM with Matrigel, the organoids similar to those when cultured on Matrigel could be cultured, in particular, it was confirmed that the culture and differentiation of pancreatic organoids were effective by increasing the expression level of pancreatic-specific markers.

### Example 14: Verification of Effects by Stirring Time and Stirring Speed of the S4) Lysing

This Example describes the experimental method in which the stirring time and speed were varied in the S4) lysing according to the present invention, and the results obtained therefrom.

### (1) Experimental Method

The experimental method was carried out based on the methods described in Example 2 and Examples 10 to 12 of the present invention.

In order to prepare dECMs with different stirring time and speed, a decellularization composition was prepared according to the method of Example 2 of the present invention.

First, in order to prepare dECM with different stirring time, the pulverized dECM prepared in Example 2 was put into a 50 mL conical tube, and 0.5 M acetic acid was added together with pepsin and lysed at a stirring speed of 330 rpm for 24 hours, 72 hours, 96 hours and 120 hours, respectively.

In addition, in order to prepare dECM with different stirring speed, the mixture was lysed at a stirring speed of 80 rpm, 150 rpm, 250 rpm, 330 rpm, 500 rpm, and 1000 rpm for 72 hours. The lysed dECM was filtered by using a cell strainer to filter out the pancreatic dECM powder that was not lysed.

Subsequently, in order to adjust the pH of the prepared decellularization composition, 10X PBS and 10 N sodium hydroxide (NaOH) were added, wherein the PBS and NaOH were stored at a refrigerated temperature before use and used in a cold state for the pH adjustment.

In addition, in order to test the organoid culture depending on the stirring speed and the stirring time, first, the pancreatic organoid was cultured according to the method of Example 10 of the present invention.

In order to encapsulate the cultured pancreatic organoid in the dECM, the pancreatic organoid was obtained with a 1000 ul tip and a pipette according to the method of Example 10 of the present invention, and collected in a conical tube. After that, the organoid was primarily disassembled by using a syringe and a needle, and then pipetting was additionally performed with a 10 ml tip and a pipette aid. The disassembled organoid was obtained by subjecting the same to centrifugation and removing the supernatant.

The number of the organoids required for this experiment and the required amount of the dECM were calculated by calculating an appropriate sub-culture ratio depending on the state of the organoid (40 ul dECM required per dome). The calculated amount of the organoid pellet was mixed with each of the dECMs prepared by applying different stirring time (24 hours, 72 hours, 96 hours, 120 hours) and stirring speed (80 rpm, 150 rpm, 250 rpm, 330 rpm, 500 rpm, 1000 rpm) and Matrigel were mixed at a ratio of 2:1, respectively, and the resulting mixtures were encapsulated(This is an organoid culture optimum condition derived from Examples 11-13 and FIGS. 9-12c of the present invention). The dECM mixed with organoids was dropped in an amount of 40 ul onto a culture plate by using a pipette to form a dome for organoid culture, which was the subject to gelation at 37°C for 30-40 minutes. After that, an organoid culture medium was added, and the added medium was replaced with new one once every 2-3 days. Four days after the encapsulation of the organoid, the culture state of the organoid was observed by the method of Examples 11 and 12 according to the present invention.

### (2) Experimental Results

The physical properties of the pancreatic dECM and the results of the organoid culture for each stirring time are shown in FIGS. 13 to 16.

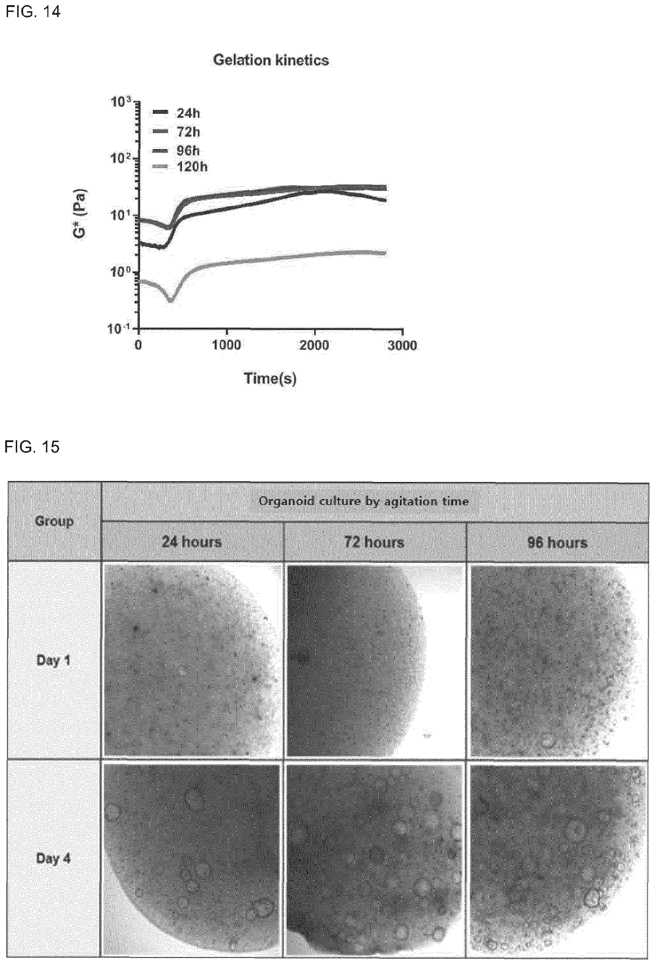

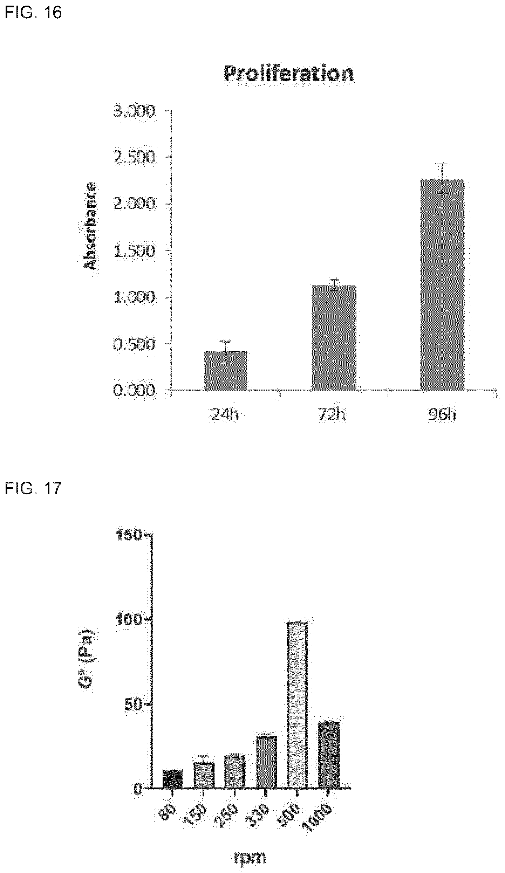

FIG. 13 is a graph showing the modulus value of the pancreatic dECM when the stirring time was changed in the S4) lysing according to an Example of the present invention; FIG. 14 is a graph showing the gelation characteristic (gelation kinetic) value of the pancreatic dECM for each stirring time; FIG. 15 is a photograph showing the pancreatic organoid culture state for each stirring time; and FIG. 16 is a graph showing the proliferation rate of the pancreatic organoid culture for each stirring time.

As shown here, the modulus value, representing the physical properties for each stirring time, was in the order of 24 h < 72 h ≒ 96 h > 120 h(24 h ≒ 120 h). Therefore, it was confirmed the physical properties were best when the stirring time was 72-96 hours (FIGS. 13 and 14).

In addition, the physical properties at 120 hours were too low, so organoid culture experiment itself was impossible. It was found that the culture tendency was improved in the order of 24 h < 72 h < 96 h in the organoid culture (FIGS. 15 and 16).

Accordingly, the present inventors learned that the conditions compatible with the organoid culture are 72-96 hours in terms of the stirring time.

In addition, the physical properties of the pancreatic dECM and the organoid culture results for each stirring speed are as shown in FIGS. 17 to 20.

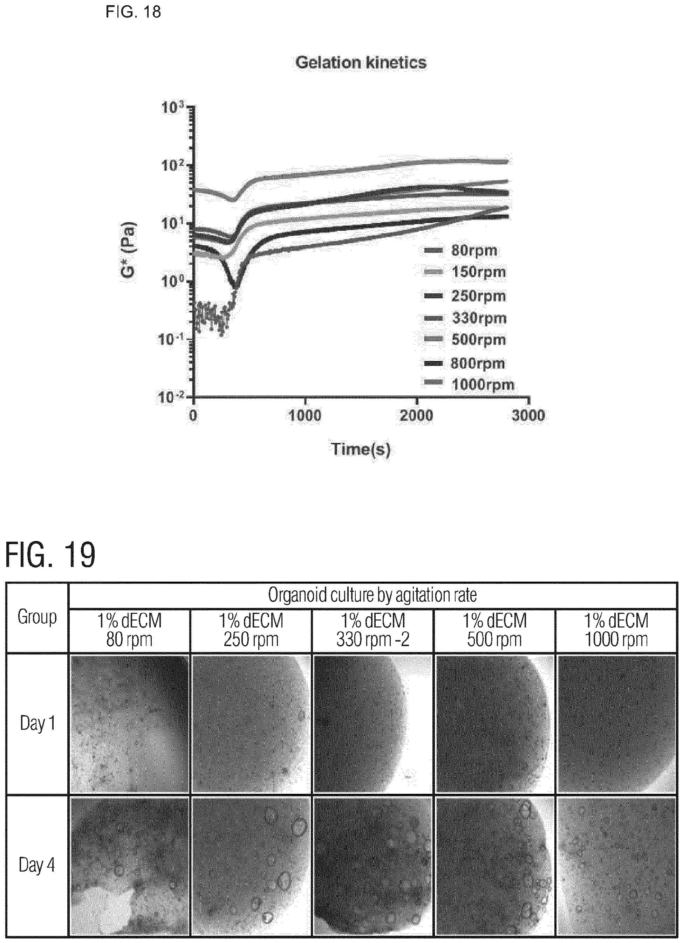

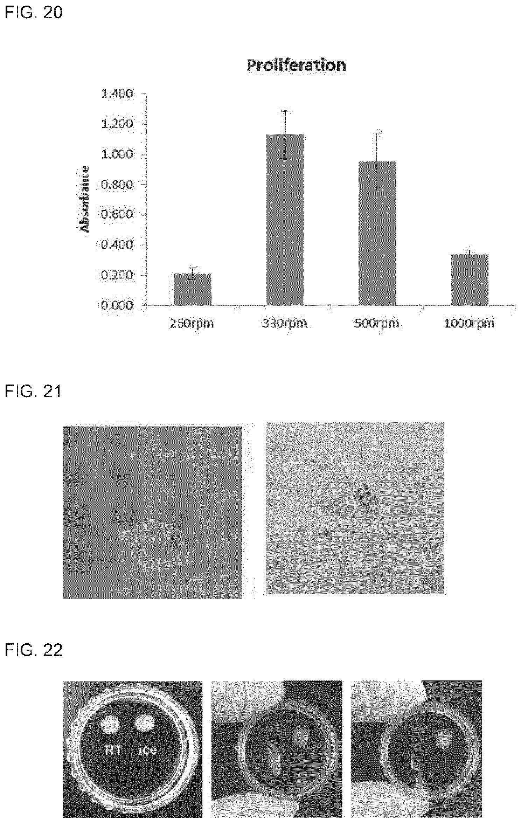

FIG. 17 is a graph showing the modulus value of the pancreatic dECM when the stirring speed was changed in the S4) lysing according to an Example of the present invention; and FIG. 18 is a graph showing the gelation characteristic (gelation kinetic) value of the pancreatic dECM for each stirring speed, FIG. 19 is a photograph showing the pancreatic organoid culture state for each stirring speed; and FIG. 20 is a graph showing the proliferation rate of the pancreatic organoid culture for each stirring speed.

As shown here, the modulus value, representing the physical properties for each stirring speed, was in the order of 80 < 150 < 250 < 330 « 500 » 1000 (330 < 1000 << 500) rpm, and it was confirmed the physical properties were best when the stirring speed was 330-500 (FIGS. 17 and 18 ).

In addition, In addition, it was confirmed that in the organoid culture, the culture efficiency was in the order of 250 « 330≒500 » 1000, and dome formation was impossible at 80 rpm.

In particular, in the dECM stirred at 80, 250, and 1000 rpm, a lot of organoid deaths occurred and no live organoid was observed, indicating that the physical properties of the organoid culture are important, and appropriate conditions could be set accordingly (FIGS. 19 and 20).

Although there was no significant difference between 250 rpm and 330 rpm in terms of physical properties by stirring speed, it can be seen that the culture at 250 rpm is significantly lower than the culture at 330 rpm in the results of organoid culture.

In addition, although the modulus values of 330 rpm and 1000 rpm do not differ significantly, and the value of 1000 rpm is slightly higher than the modulus value of 330 rpm, the results of organoid culture under agitation at 1000 rpm are remarkably low. Therefore, it can be seen that when the decellularization composition is put into solution, even a very slight difference in rpm can have a great effect on organoid culture.

Therefore, the present inventors learned that the conditions compatible with the organoid culture are 330-500 rpm in terms of the stirring speed.

### Example 15: Verification of Effect of Each Condition of the S5) adjusting pH

This Example describes the experimental method in which the implementation conditions and temperature were varied in the S5) adjusting pH according to the present invention, and the results obtained therefrom.

### (1) Experimental Method

The experimental method was carried out based on the method described in Example 2 of the present invention.

In order to prevent gelation of the pancreatic dECM lysate during the pH adjustment process, the pH adjustment process was performed while the tube containing the lysate was kept in ice.

FIG. 21 is a photograph for explaining the process of performing the S5) adjusting pH on ice according to an Example of the present invention. In other words, as shown in FIG. 21 , the pH adjustment process was performed in a state in which the container containing the decellularization composition according to the present invention was completely submerged in ice. As a control, the pH adjustment process was performed while the container containing the decellularization composition was placed at room temperature (about 26°C).

In the pH adjustment process, 10X PBS and 10 N sodium hydroxide (NaOH) were added, and the PBS and NaOH were stored at a refrigeration temperature (1°C to 10°C) in advance before use and used in a cold state for pH adjustment.

When the pH was adjusted to be neutral, a dome is made by taking the required amount, and gelation was performed at 37°C (using an incubator) for 30 minutes (see FIGS. 22, 23 and 24) .

Then, the complex modulus (G*) value of the gelated decellularization composition was measured to evaluate the physical properties.

### (2) Experimental Results

The physical properties of the pancreatic dECM lysate for each condition of the pH adjustment process are as shown in FIGS. 22 to 25.

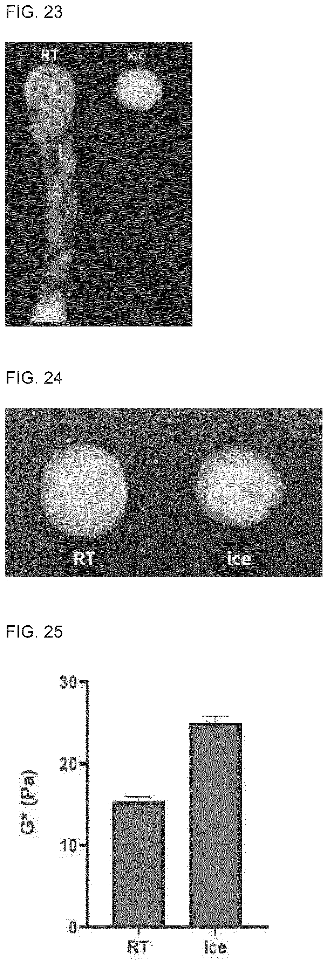

FIGS. 22 to 24 are photographs showing the gelation state of the composition for which the S5) adjusting pH was performed on ice according to an Example of the present invention; and FIG. 25 is a graph showing the modulus value of the composition for which the S5) adjusting pH was performed on ice according to an Example of the present invention.

As shown here, when the pH adjustment process was performed on ice as in the present invention, it was found that gelation of the decellularization composition was prevented, and homogeneous hydrogelation was achieved. This facilitates not only the subsequent pipetting and encapsulation but also the organoid culture.

In comparison, when the pH adjustment process was performed at room temperature (RT), a portion of the dECM was partially gelated and aggregated, and as time passed, the separation of the water layer and the decellularization composition occurred, resulting in a non-homogeneous state (see FIGS. 22 and 23).

In addition, after the pH adjustment step, the decellularization composition was gelated at 37°C for 30 minutes in the same manner as the organoid culture condition, and then the hydrogels made under each pH adjustment condition were observed. The results also showed that the separation of the water layer and the gelated decellularization composition occurred more significantly in the hydrogel of which pH was adjusted at room temperature (RT), confirming that the hydrogel fails to maintain the gel stably and so is incompatible with the organoid culture (see FIG. 24).

According to the results, the decellularization composition of which pH was adjusted at room temperature was difficult to perform pipetting when encapsulating the organoid, and the dome was not maintained but was broken during the culturing of the encapsulated organoid or the culture itself was difficult because the organoid was not encapsulated homogeneously (FIG. 22, 23 and 24).

In other words, the decellularization composition of which pH was adjusted on ice according to the present invention was able to form a homogeneous gel during the gelation process, but the decellularization composition of which pH was adjusted at room temperature (RT) was partially gelated and aggregated, and the dome was not maintained but was broken, exhibiting low physical properties (complex modulus (G*)) (see FIG. 25).

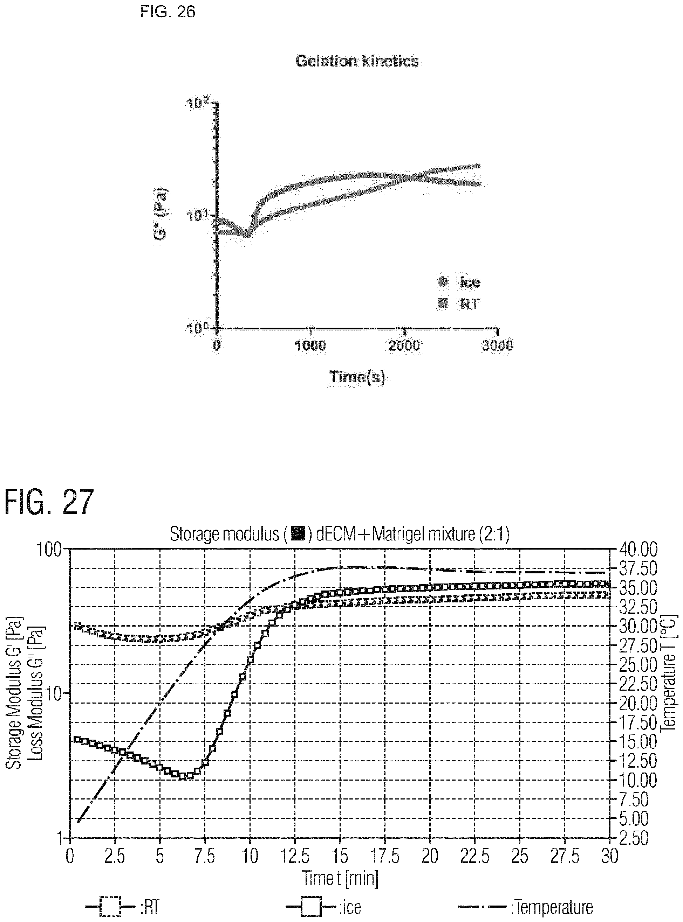

In addition, FIG. 26 is a graph showing the gelation kinetic value of the composition for which the S5) adjusting pH was performed on ice according to an Example of the present invention.

The decellularization composition of which pH was adjusted on ice according to the present invention showed a clear increase in the complex modulus (G*) value during temperature change (4-37°C), whereas the decellularization composition of which pH was adjusted at room temperature (RT) already showed a liner change in G * value, this means that when the pH is already adjusted at room temperature, partial gelation has occurred and gelation has occurred. These results indicate that when the pH is adjusted at room temperature (RT), difficulty arises in pipetting when encapsulating organoids, and it affects dome formation and maintenance, making organoid culture itself difficult (see FIG. 26).

In addition, under the dECM + matrigel (2: 1) mixed condition selected as the optimum condition for organoid culture according to Examples 10-13 of the present invention, when analyzing the change in physical properties according to the increase in temperature, it was confirmed that the difference in initial physical properties between when pH adjustment was made at room temperature (RT) and when made on ice was larger than when dECM alone (see FIG. 27).

In addition, the decellularization composition in which the pH was adjusted on ice showed a pattern of changing from a sol state to a gel state, but almost no phase change occurred when the pH was adjusted at room temperature (RT). It was found that it was difficult to perform and therefore not suitable for organoid culture (see FIG. 27).

In the above, the applicant has described preferred Examples of the present invention, but these Examples are only one Example that implements the technical principles of the present invention, and any changes or modifications should be construed as being within the scope of the present invention as along as the technical principles of the present invention are implemented.

## Claims

1. A method for preparing a composition for culturing pancreatic organoids, the method comprising:
S1) decellularizing pancreatic tissue;
S2) lyophilizing the decellularized tissue;
S3) pulverizing the lyophilized tissue;
S4) lysing the pulverized tissue by adding a proteopeptic enzyme and an acid and stirring at a speed of 330 rpm to 500 rpm for 72 to 96 hours; and
S5) adjusting the pH by adding a basic solution to the lysate.

2. The method for preparing a composition for culturing pancreatic organoids according to claim 1, **characterized in that** the S4) is lysing the pulverized tissue by adding a proteopeptic enzyme and an acid and stirring at a speed of 330 rpm for 72 to 96 hours.

3. The method for preparing a composition for culturing pancreatic organoids according to claim 1, **characterized in that** the S4) is lysing the pulverized tissue by adding a proteopeptic enzyme and an acid and stirring at a speed of 330 rpm to 500 rpm for 96 hours.

4. The method for preparing a composition for culturing pancreatic organoids according to claim 1, **characterized in that** the S4) is lysing the pulverized tissue by adding a proteopeptic enzyme and an acid and stirring at a speed of 330 rpm for 96 hours.

5. A method for preparing a composition for culturing pancreatic organoids, the method comprising:
S1) decellularizing pancreatic tissue;
S2) lyophilizing the decellularized tissue;
S3) pulverizing the lyophilized tissue;
S4) lysing the pulverized tissue by adding a proteopeptic enzyme and an acid; and
S5) adjusting the pH by adding a basic solution to the lysate,
**characterized in that** the S5) is performed within a range of 2°C to 7°C to prevent gelation.

6. A method for preparing a composition for culturing pancreatic organoids, the method comprising:
S1) decellularizing pancreatic tissue;
S2) lyophilizing the decellularized tissue;
S3) pulverizing the lyophilized tissue;
S4) lysing the pulverized tissue by adding a proteopeptic enzyme and an acid; and
S5) adjusting the pH by adding a basic solution to the lysate,
**characterized in that** the S5) is performed on ice to prevent gelation.

7. The method for preparing a composition for culturing pancreatic organoids according to claim 6, **characterized in that** performing on ice is carried out by inserting a container containing the lysate into ice and then adjusting the pH by adding a basic solution.

8. The method for preparing a composition for culturing pancreatic organoids according to claim 7, **characterized in that** the basic solution is stored at a refrigeration temperature within a range of 1°C to 10°C in advance before use.

9. A composition for culturing pancreatic organoids, the composition prepared by a method according to any one of claims 1-8.

10. The composition for culturing pancreatic organoids according to claim 9, **characterized in that** the composition further comprising Matrigel.

11. A pancreatic organoid culture method comprising culturing a pancreatic organoid in the composition for culturing pancreatic organoids according to claim 9.

12. An extracellular matrix scaffold for culturing organoids, the scaffold prepared with the composition for culturing pancreatic organoids according to claim 9.
